# FASCICULE DE BREVET EUROPEEN

(11) **EP 0 531 224 B1**
(45) Date de publication et mention de la délivrance du brevet: **04.10.1995**
(21) Numéro de dépôt: 92402429.2
(22) Date de dépôt: 04.09.1992
(51) Int. Cl.: A61K 7/48, A61K 7/32

(54) **Composition cosmétique sous forme d'un gel solide**
Kosmetisches Mittel in Form eines festen Gels
Solid gel-like cosmetic composition

(30) Priorité: 06.09.1991 FR 9111062
(43) Date de publication de la demande: 10.03.1993
(73) Titulaire: L'OREAL, 75008 Paris (FR)
(72) Inventeur: Kauffmann, Myriam, F-69006 Lyon (FR); Gregoire, Nathalie, F-92330 Sceaux (FR); Quemin, Eric, F-93420 Villepinte (FR)
(74) Mandataire: Stalla-Bourdillon, Bernard

(56) Documents cités:
- EP-A- 0 260 030
- US-A- 4 717 498

## Description

La présente invention a pour objet une composition cosmétique pour une application sur la peau se présentant sous forme d'un gel solide transparent ou translucide. Plus particulièrement, l'invention se rapporte à des produits sous forme de pains solides ou de sticks ayant des propriétés hydratantes pour une application sur la peau.

Parmi les compositions cosmétiques se présentant sous forme de sticks transparents, on peut notamment citer les déodorants. Leur conservation soulève un certain nombre de difficultés car ils ont tendance à se dessécher et à s'opacifier au cours du stockage. En effet, par évaporation des ingrédients volatils, ils subissent des phénomènes de contraction les déformant et les rendant très friables. Par évaporation des ingrédients volatils, ils peuvent également devenir opaques.

Parmi les ingrédients volatils, l'éthanol est largement utilisé et sa présence peut par ailleurs provoquer une sensation desséchante sur la peau en la délipidant et la déshydratant.

L'utilisation d'alcool inférieur tel que l'éthanol est rendu nécessaire pour solubiliser certains des ingrédients et en particulier l'agent gélifiant tel que par exemple le dibenzylidène sorbitol.

On peut à ce sujet citer comme état de la technique, les brevets US 4.154.816 et 4.346.079 ainsi que la demande de brevet européen 260 030.

On a maintenant découvert de façon surprenante et inattendue qu'il était possible d'obtenir des gels non-alcooliques sous forme de pains ou de sticks transparents en utilisant comme agent gélifiant un dibenzylidène-ose en association avec un agent durcisseur du type sulfosuccinate.

Les sulfosuccinates ont été décrits dans la littérature comme constituant des agents tensioactifs ayant des propriétés peu irritantes pour la peau et les cheveux et ayant la propriété d'abaisser le pouvoir irritant de tensioactifs anioniques tels que le lauryléthersulfate de sodium.

La présente invention a donc pour objet à titre de produit industriel nouveau un gel cosmétique solide, non-alcoolique, sous forme d'un pain ou d'un stick, ce gel contenant :
- de 65 à 99 % en poids d'un polyol,
- de 0,1 à 5 % en poids d'un dibenzylidène-ose,
- de 0,1 à 5 % en poids d'un agent durcisseur du type sulfosuccinate, et
- de 0,5 à 40 % en poids d'eau.

L'emploi selon l'invention d'un agent durcisseur du type sulfosuccinate en association avec un dibenzylidène-ose permet d'obtenir par un effet de synergie, des gels rigidifiés non-alcooliques.

Les gels solides selon l'invention ne présentent aucun des inconvénients de l'art antérieur dans la mesure où ils sont stables au stockage, ne s'effritent pas, restent transparents et ne sont pas irritants.

L'emploi d'un polyol tel que par exemple la glycérine confère aux gels d'excellentes propriétés hydratantes.

Selon l'invention, le polyol est de préférence présent à une concentration comprise entre 65 et 80 % en poids et est choisi parmi la glycérine, la polyglycérine, les polyéthylèneglycols tels que les composés de formule 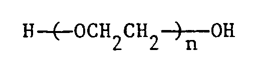
avec n compris entre 6 et 115000. Parmi ces derniers, on peut citer de préférence les produits connus sous les dénominations CTFA tels que PEG-6, PEG-8, PEG-12, PEG-6-32, PEG-20, PEG-150, PEG-7M, PEG-12M et PEG-115M. Sont plus particulièrement préférés les produits vendus sous les dénominations "Polyéthylène glycol 300" et "Polyéthylène glycol 20000" vendus par la Société HOECHST, "Polyéthylène glycol 400", "Polyéthylène glycol 600" et "Polyéthylène glycol 1500" vendus par la Société BP (British Petroleum), "Carbowax 1000", "Carbowax 8000", "Polyox WSR N-750" et "Polyox coagulant" vendus par la Société UNION CARBIDE.

Selon l'invention, le polyol peut être également le propylène glycol, le sorbitol ou un étheralcool tel que les éthers de glycol. Parmi ces derniers, on peut citer plus particulièrement le méthyléther de polypropylène glycol (à 2 groupements propylène oxy) et le méthyléther de polyéthylène glycol (à 2 groupements éthylène oxy) vendus sous les dénominations de "DOWANOL DPM" et "DOWANOL DM" par la Société DOW CORNING.

Ces polyols sont utilisés selon l'invention comme solvant du dibenzylidène-ose qui constitue l'agent gélifiant.

Comme dibenzylidène-ose, on peut utiliser selon l'invention les composés répondant à la formule générale suivante : 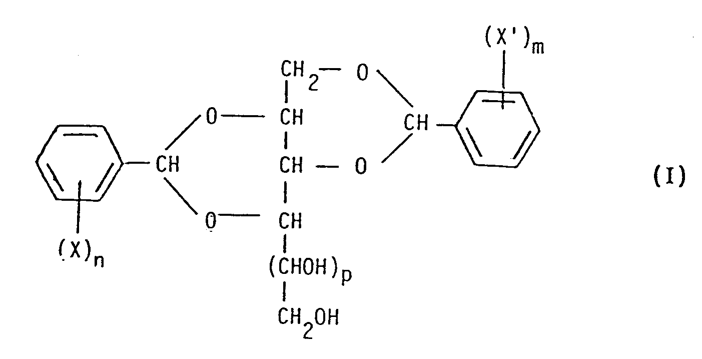
dans laquelle :
X et X', identiques ou différents, représentent un atome d'hydrogène, un atome d'halogène, un alkyle en C₁-C₄, un alcoxy en C₁-C₄ ou un groupe carboxyle,
m et n représentent un nombre entier de 0 à 5, et
p est 0 ou 1.

Parmi les composés de formule (I), on peut notamment mentionner le dibenzylidène sorbitol, le dibenzylidène-ribitol et le dibenzylidène-xylitol.

Ces composés permettent de conférer à la composition sa rigidité ainsi que son caractère transparent ou translucide.

Ces composés sont connus et leur préparation a notamment été décrite dans la demande de brevet européen n° 319.917.

L'agent durcisseur de type sulfosuccinate est de préférence présent à une concentration comprise entre 1 et 2 % en poids et est :
- soit une silicone sulfosuccinate de formule (II) : 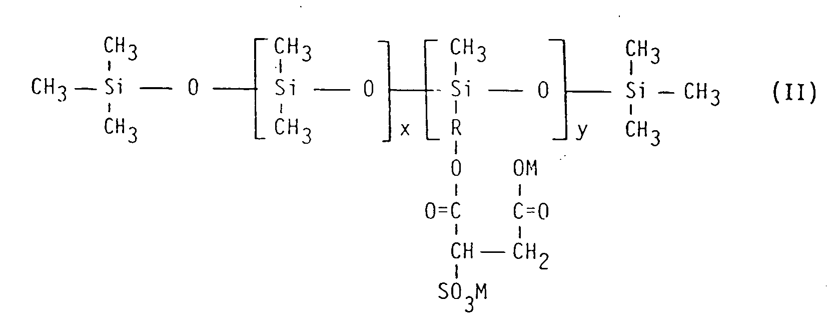 dans laquelle :
   R représente un radical divalent : 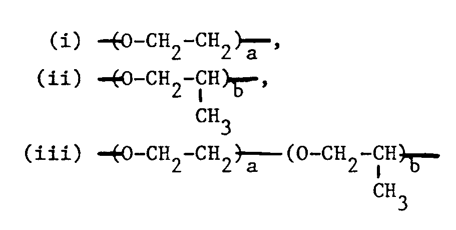 a et b étant compris entre 0 et 30
   x et y étant tel que le poids moléculaire est compris entre 700 et 1600, et
   M est un métal alcalin tel que le sodium ou le potassium ou un groupe ammonium,
- soit un sulfosuccinate à chaîne stérol de formule (III) : 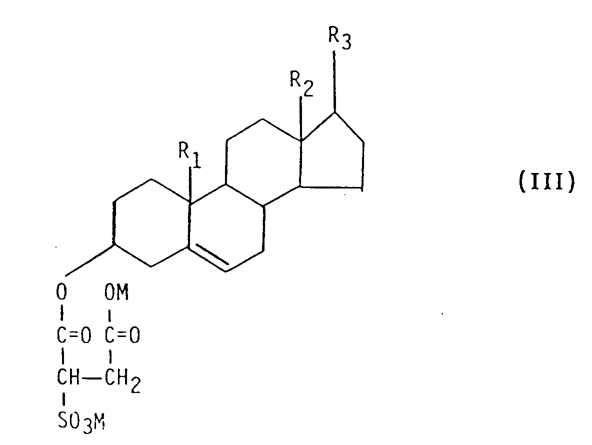 dans laquelle :
   R₁ et R₂, identiques ou différents, représentent un atome d'hydrogène ou le radical -CH₃,
   R₃ représente un alkyle en C₁-C₂₀, et
   M est un métal alcalin tel que le sodium ou le potassium ou un groupe ammonium.

Parmi les silicones sulfosuccinates de formule (II), on peut notamment citer celles décrites dans le brevet US 4.849.127 et en particulier le produit vendu sous la dénomination de "MACKANATE DC30" par la Société MAC INTYRE CHEMICAL COMPANY.

Parmi les sulfosuccinates à chaîne stérol de formule (III), on peut notamment citer le β-sitostérol sulfosuccinate, composé pour lequel R₁ et R₂ représentent CH₃ et R₃ est un radical alkyle de formule 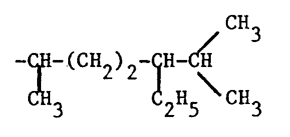
Les agents durcisseurs de type sulfosuccinate selon les formules (II) et (III) ont un effet synergique en présence du dibenzylidène-ose sur la dureté. Lorsqu'ils sont employés seuls, ils ne confèrent aucune viscosité au milieu qui reste liquide. Par contre, lorsqu'ils sont ajoutés au dibenzylidène-ose, même en faible quantité, ils permettent de multiplier la résistance mécanique du gel par un facteur de 2 à 10.

La dureté du gel cosmétique solide selon l'invention est généralement comprise entre 1,5 et 5 Newton (N).

Le gel cosmétique selon l'invention peut également contenir d'autres ingrédients permettant d'améliorer sa transparence, son toucher cosmétique, ou qui facilite sa fabrication. Ainsi, les sticks peuvent comprendre des co-solvants du dibenzylidène-ose comme la butyrolactone, le propylène carbonate ou la pyrrolidone. On peut également incorporer des humectants tels que le sorbitol ou l'urée.

L'introduction d'huiles émollientes ou lubrifiantes est facilitée par la présence de l'agent durcisseur de type sulfosuccinate, ce dernier conférant en outre des propriétés émulsionnantes à la composition.

Parmi les huiles, on peut citer les huiles minérales telles que l'huile de vaseline ou l'isohexadécane, les huiles végétales comme l'huile d'abricot ou l'huile de jojoba, des huiles de silicone, des huiles perfluorées, les huiles essentielles et tous les lipides liquides à la température de préparation du gel cosmétique solide.

Il est également possible d'introduire des agents moussants ou des tensioactifs détergents habituellement utilisés en cosmétique.

Enfin, on peut incorporer des actifs, des parfums, des aromatisants, des agents conservateurs, des antioxydants, des filtres ainsi que des colorants qui n'altèrent ni le toucher ni la transparence de la composition.

On peut également introduire des charges, des poudres minérales telles que du talc, de la silice, de l'oxyde de titane, ou un oxyde métallique ainsi que tout autre produit habituellement utilisé en cosmétique et en dermo-pharmacie. Ces charges opacifient ou colorent la composition et peuvent lui conférer certaines propriétés, par exemple un effet exfoliant.

Bien que l'invention ait été plus particulièrement décrite en faisant référence à des pains ou des sticks, il va de soi qu'elle s'applique également à toute autre forme solide utilisée en cosmétique comme par exemple les crayons à mine aqueuse.

Les composés actifs peuvent être du type hydrosoluble, liposoluble ou soluble dans les polyols, par exemple des vitamines ou acides aminés, des actifs amincissants pour le corps et le visage, tels que la caféine et ses dérivés, des actifs antiacnéiques, antimycosiques ou antiseptiques tels que le thiolanediol, des actifs dépigmentants tels que l'acide kojique, des actifs hydratants tels que l'acide lactique ou l'urée ou encore l'acide hyaluronique. De façon générale, tous les actifs utilisables en cosmétique et en dermo-pharmacie peuvent être introduits sous réserve toutefois qu'ils ne soient pas dégradés par la chaleur lors de la fabrication.

La présente invention a également pour objet le procédé de fabrication des sticks ou pains solides tels que définis ci-dessus.

Ce procédé comprend les étapes consistant :
(a) à dissoudre, dans un réacteur muni d'un condensateur, à une température comprise entre 130 et 160°C, le dibenzylidène-ose dans le polyol,
(b) à introduire dans le réacteur le mélange d'eau et d'agent durcisseur de type sulfosuccinate,
(c) à introduire, si nécessaire, les composés actifs et autres ingrédients thermosensibles, et
(d) à couler, dans des moules appropriés, la composition et à laisser refroidir.

On va maintenant donner à titre d'illustration et sans aucun caractère limitatif, plusieurs exemples de gels cosmétiques solides.

### EXEMPLE 1 : Démaquillant

| | |
|---|---|
| - Polyéthylène glycol 400 | 37 % |
| - Glycérine | 30 % |
| - Dibenzylidène sorbitol | 3 % |
| - "Mackanate DC30" (MAC INTYRE) solution aqueuse à 30 % de matière active | 5 % |
| - Décylpolyglucoside à 50 % dans l'eau "APG 300" (HENKEL) | 10 % |
| - Alginate de propylène glycol | 0,15 % |
| - Eau | 14,85 % |

On peut couler ce démaquillant sous forme d'un pain transparent, qui a une dureté de 2N. Ce pain est très hydratant, mousse à l'application, se rince très bien à l'eau et laisse un film doux sur la peau.

### EXEMPLE 2 : "Savon" à raser sans savon

| | |
|---|---|
| - Polyéthylène glycol 400 | 37 % |
| - Glycérine | 30 % |
| - Dibenzylidène sorbitol | 3 % |
| - "Mackanate DC30" (MAC INTYRE) | 10 % |
| - Polydiméthylsiloxane "Abil 10" (GOLDSCHMIDT) | 1 % |
| - "APC 300" (HENKEL) | 10 % |
| - Eau | 9 % |

Ce savon a une dureté de 2,5 N.

Il mousse et lubrifie à la fois. Il est très hydratant et laisse la peau douce.

### EXEMPLE 3 : Crayon réparateur pour les lèvres

| | |
|---|---|
| - Polyéthylène glycol 400 | 51 % |
| - Glycérine | 32 % |
| - Urée | 3 % |
| - Dibenzylidène sorbitol | 4 % |
| - "Mackanate DC30" (MAC INTYRE) | 5 % |
| - Eau | 5 % |

Ce crayon a une dureté voisine de 3,5 N.

Il est hydratant et a un bon effet pour le soin des lèvres.

### EXEMPLE 4 : Pain hydratant pour le bain, sans savon

| | |
|---|---|
| - Polyéthylène glycol 400 | 37 % |
| - Glycérine | 30 % |
| - Dibenzylidène sorbitol | 3 % |
| - β-sitostérol sulfosuccinate de sodium | 1,5 % |
| - Eau | 28,5 % |
| - Parfum | qs |

Ce pain a une dureté d'environ 3 N.

### EXEMPLE 5 : Stick amincissant

| | |
|---|---|
| - Polyéthylène glycol 400 | 37 % |
| - Glycérine | 30 % |
| - Dibenzylidène sorbitol | 3 % |
| - "Mackanate DC30" (MAC INTYRE) | 5 % |
| - Eau | 19,24 % |
| - Caféine | 3 % |
| - Acide salicylique | 1,26 % |
| - Triéthanolamine | 1,5 % |

La dureté de ce stick est d'environ 2 N.

Ce stick, appliqué en massage, constitue un excellent amincissant.

### EXEMPLE 6 : Stick dépigmentant

| | |
|---|---|
| - Polyéthylène glycol 400 | 37 % |
| - Glycérine | 14 % |
| - Polyglycérine 500 | 14 % |
| - Dibenzylidène sorbitol | 4 % |
| - "Mackanate DC30" (MAC INTYRE) | 5 % |
| - Tampon acétate de pH 4,7 | 24 % |
| - Acide kojique | 1 % |

Ce stick a une dureté d'environ 2,5 N.

Il est facile à appliquer sur la peau et permet une bonne dépigmentation des taches.

## Revendications

1. Gel cosmétique solide, non alcoolique, sous forme d'un pain ou d'un stick caractérisé par le fait qu'il contient :
- de 65 à 99 % en poids d'un polyol,
- de 0,1 à 5 % en poids d'un dibenzylidène-ose,
- de 0,1 à 5 % en poids d'un agent durcisseur du type sulfosuccinate, et
- de 0,5 à 40 % en poids d'eau.

2. Cet selon la revendication 1, caractérisé par le fait que le polyol est présent à une concentration comprise entre 65 et 80 % en poids par rapport au poids total du gel.

3. Gel selon la revendication 1 ou 2, caractérisé par le fait que le polyol est choisi parmi la glycérine la polyglycérine tes polyéthylène glycols, le propylène glycol, le sorbitol et les étheralcools.

4. Gel selon la revendication 1, caractérisé par le fait que le dibenzylidène-ose répond à la formule générale suivante : 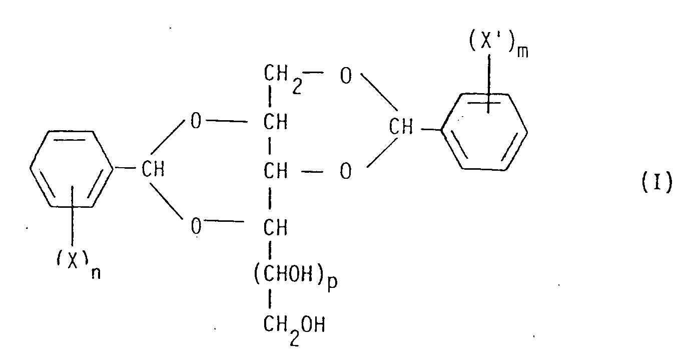 dans laquelle :
X et X', identiques ou différents, représentent un atome d'hydrogène, un atome d'halogène, un alkyle en C₁-C₄, un alcoxy en C₁-C₄ ou un groupe carboxyle,
m et n représentent un nombre entier de 0 à 5, et
p est 0 ou 1.

5. Gel selon la revendication 1 ou 4, caractérisé par le fait que le dibenzylidène-ose est choisi parmi le dibenzylidène-sorbitol, le dibenzylidène-ribitol et le dibenzylidène-xylitol.

6. Gel selon la revendication 1, caractérisé par te fait que l'agent durcisseur de type sulfosuccinate est un sulfosuccinate de formule : 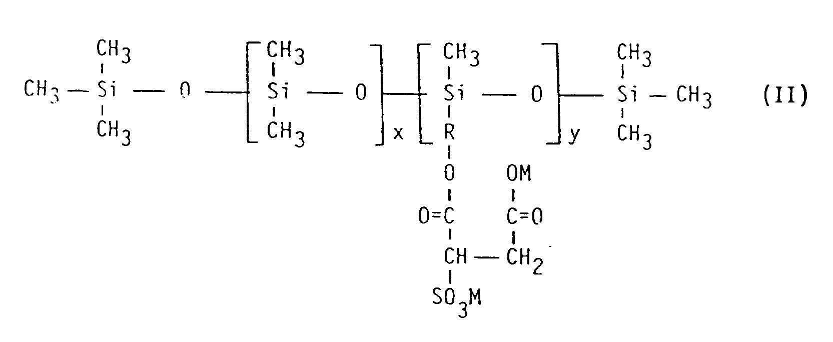 dans laquelle :
R représente un radical divalent : 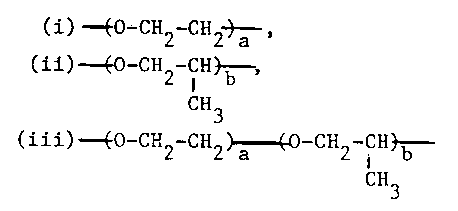 a et b étant compris entre 0 et 30
x et y étant tel que te poids moléculaire est compris entre 700 et 1600, et
M est un métal alcalin tel que le sodium ou te potassium ou un groupe ammonium.

7. Gel selon ta revendication 1, caractérisé par le fait que l'agent durcisseur de type sulfosuccinate est un sulfosuccinate à chaîne stérol de formule : 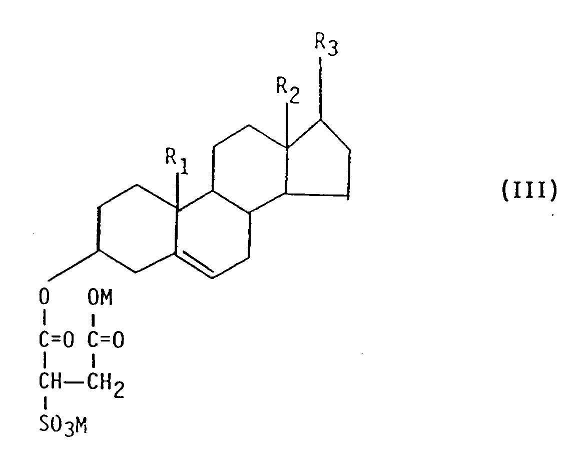 dans laquelle :
R₁ et R₂, identiques ou différents, représentent un atome d'hydrogène ou le radical -CH₃,
R₃ représente un alkyle en C₁-C₂₀, et
M est un métal alcalin tel que le sodium ou le potassium ou un groupe ammonium.

8. Gel selon l'une quelconque des revendications précédentes, caractérisé par le fait qu'il présente une dureté comprise entre 2 et 5 Newton (N).

9. Procédé de préparation des gels selon l'une quelconque des revendications 1 à 8, caractérisé par le fait qu'il consiste :
(a) à dissoudre, dans un réacteur muni d'un condensateur, à une température comprise entre 130 et 160°C, le dibenzylidène-ose dans le polyol,
(b) à introduire dans le réacteur le mélange d'eau et d'agent durcisseur de type sulfosuccinate,
(c) à introduire, si nécessaire, les composés actifs et autres ingrédients thermosensibles, et
(d) à couler, dans des moules appropriés, la composition et à laisser refroidir.

## Claims

1. Non-alcoholic solid cosmetic gel in the form of a cake or a stick, characterized in that it contains:
- from 65 to 99 % by weight of a polyol,
- from 0.1 to 5 % by weight or a dibenzylidenemonosaccharide,
- from 0.1 to 5 % by weight of a hardening agent of the sulphosuccinate type, and
- from 0.5 to 40 % by weight of water.

2. Gel according to Claim 1, characterized in that the polyol is present at a concentration of between 65 and 80 % by weight relative to the total weight of the gel.

3. Gel according to Claim 1 or 2, characterized in that the polyol is chosen from glycerol, polyglycerol, polyethylene glycols, propylene glycol, sorbitol and ether alcohols.

4. Gel according to Claim 1, characterized in that the dibenzylidene-monosaccharide corresponds to the following general formula: 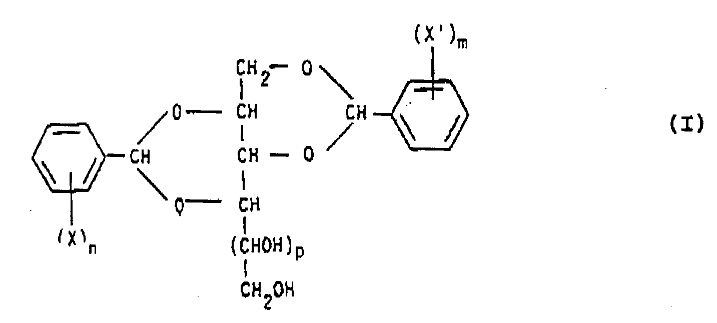 in which:
X and X', which are identical or different, represent a hydrogen atom, a halogen atom, a C₁-C₄ alkyl, a C₁-C₄ alkoxy or a carboxyl group,
m and n represent an integer from 0 to 5, and
p is 0 or 1.

5. Gel according to Claim 1 or 4, characterized in that the dibenzylidene-monosaccharide is chosen from dibenzylidene-sorbitol, dibenzylidene-ribitol and dibenzylidene-xylitol.

6. Gel according to Claim 1, characterized in that the sulphosuccinate type hardening agent is a sulphosuccinate of formula: 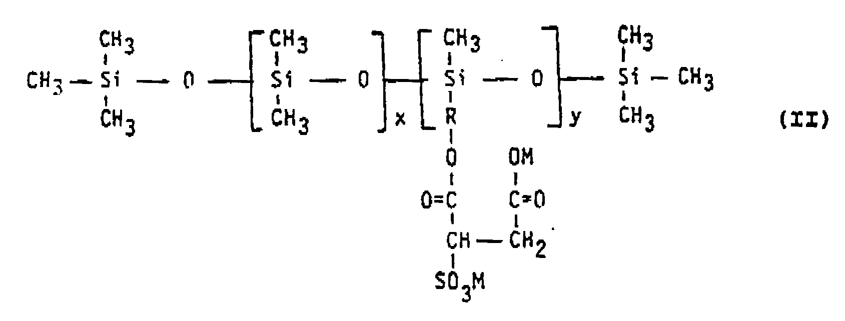 in which:
R represents a divalent radical: 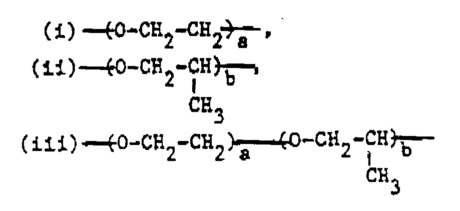 a and b being between 0 and 30
x and y being such that the molecular weight is between 700 and 1600, and
M is an alkali metal such as sodium or potassium or an ammonium group.

7. Gel according to Claim 1, characterized in that the sulphosuccinate type hardening agent is a sterol chain-containing sulphosuccinate of formula: 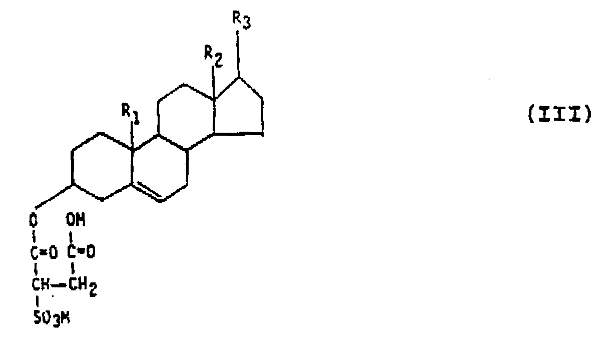 in which:
R₁ and R₂, which are identical or different, represent a hydrogen atom or the -CH₃ radical,
R₃ represents a C₁-C₂₀ alkyl, and
M is in alkali metal such as sodium or potassium or an ammonium group.

8. Gel according to any one of the preceding claims, characterized in that it has a hardness of between 2 and 5 Newton (N).

9. Process for preparing gels according to any one of Claims 1 to 8, characterized in that it consists:
(a) in dissolving, in a reactor provided with a condenser, at a temperature of between 130 and 160°C, the dibenzylidene-monosaccharide in the polyol,
(b) in introducing the mixture of water and sulphosuccinate type hardening agent into the reactor,
(c) in introducing, if necessary, the active compounds and other heat-sensitive ingredients, and
(d) in casting the composition in appropriate moulds and in allowing it to cool.

## Patentansprüche

1. Nichtalkoholisches, festes kosmetisches Gel in Kuchen- oder Stick-Form, dadurch gekennzeichnet, daß es
- 65 bis 99 Gew.-% eines Polyols,
- 0,1 bis 5 Gew.-% einer Dibenzylidenose,
- 0,1 bis 5 Gew.-% eines Härters vom Sulfosuccinat-Typ und
- 0,5 bis 40 Gew.-% Wasser enthält.

2. Gel nach Anspruch 1, dadurch gekennzeichnet, daß das Polyol in einer Konzentration zwischen 65 und 80 Gew.-%, bezogen auf das Gesamtgewicht des Gels, vorliegt.

3. Gel nach Anspruch 1 oder 2, dadurch gekennzeichnet, daß das Polyol aus Glycerin, Polyglycerin, Polyethylenglykolen, Propylenglykol, Sorbit und Estheralkoholen ausgewählt ist.

4. Gel nach Anspruch 1, dadurch gekennzeichnet, daß die Dibenzylidenose folgender allgemeiner Formel entspricht: 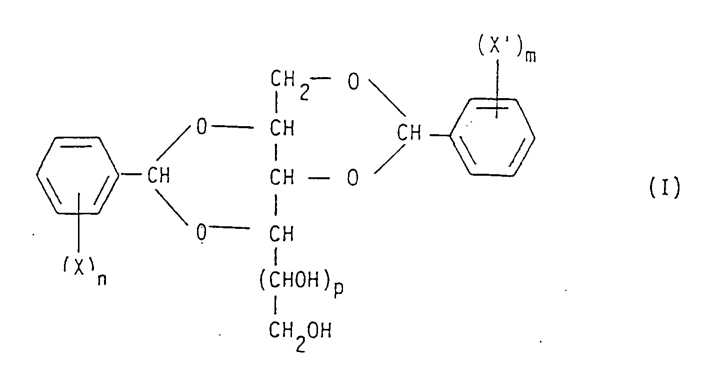 in der
X und X' gleich oder verschieden sind und ein Wasserstoffatom, ein Halogenatom, ein C₁-C₄-Alkyl, C₁-C₄-Alkoxy oder eine Carboxylgruppe bedeuten,
m und n eine Zahl zwischen 0 bis 5 darstellen und
p 0 oder 1 bedeutet.

5. Gel nach Anspruch 1 oder 4, dadurch gekennzeichnet, daß die Dibenzylidenose aus Dibenzylidensorbit, Dibenzylidenribit und Dibenzylidenxylit ausgewählt ist.

6. Gel nach Anspruch 1, dadurch gekennzeichnet, daß das Härtungsmittel vom Sulfosuccinat-Typ ein Sulfosuccinat der Formel: 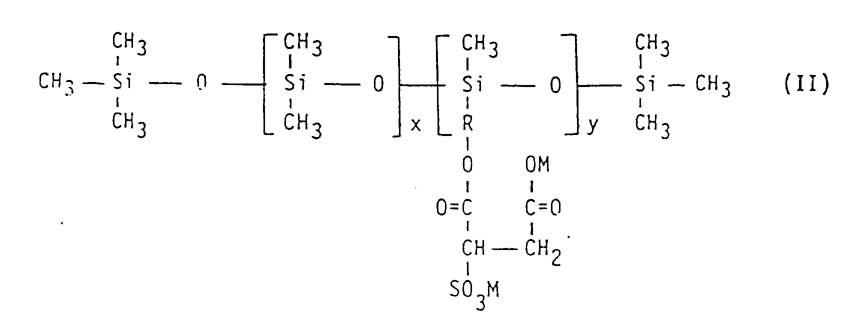 ist, in der:
R einen zweiwertigen Rest bedeutet: 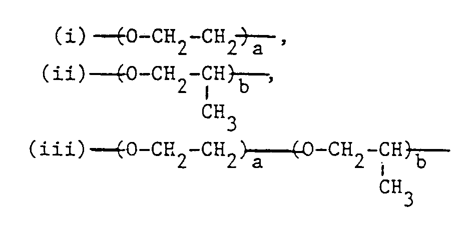 a und b zwischen 0 und 30 liegen,
x und y einen Wert aufweisen, daß das Molekulargewicht zwischen 700 und 1.600 ist, und
M ein Alkalimetall wie Natrium oder Kalium oder eine Ammoniumgruppe bedeutet.

7. Gel nach Anspruch 1, dadurch gekennzeichnet, daß das Härtungsmittel vom Sulfosuccinat-Typ ein Sulfosuccinat mit einer Sterol-Kette folgender Formel ist: 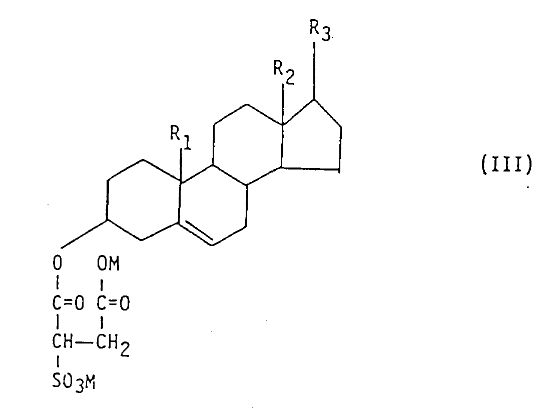 in der:
R₁ und R₂ gleich oder verschieden sind und ein Wasserstoffatom oder den Rest -CH₃ bedeuten,
R₃ ein C₁-C₂₀-Alkyl bedeutet und
M ein Alkalimetall wie Natrium oder Kalium oder eine Ammonium-Gruppe bedeutet.

8. Gel nach einem der vorstehenden Ansprüche, dadurch gekennzeichnet, daß es eine Härte zwischen 2 und 5 Newton (N) aufweist.

9. Verfahren zur Herstellung von Gelen nach einem der Ansprüche 1 bis 8, dadurch gekennzeichnet, daß man:
(a) in einem mit einem Kondensator ausgerüsteten Reaktor bei einer Temperatur zwischen 130 und 160°C die Dibenzylidenose in Polyol auflöst,
(b) in den Reaktor das Gemisch aus Wasser und dem Härtungsmittel vom Sulfosuccinat-Typ einführt,
(c) gegebenenfalls die aktiven Verbindungen und andere thermosensible Ingredienzien einführt und
(d) die Zusammensetzung in geeignete Formen eingießt und abkühlen läßt.
